(19)

Europäisches
Patentamt
European
Patent Office
Office européen
des brevets

(11)     EP 4 524 881 A1

(12)                                    **EUROPEAN PATENT APPLICATION**

(43) Date of publication:
   **19.03.2025   Bulletin 2025/12**

(21) Application number: **23196946.0**

(22) Date of filing: **12.09.2023**

(51) International Patent Classification (IPC):
   *G06T 7/00* (2017.01)      *G06T 7/11* (2017.01)
   *G16H 30/40* (2018.01)     *G16H 50/20* (2018.01)

(52) Cooperative Patent Classification (CPC):
   **G06T 7/0012; G06T 7/11; G16H 30/40; G16H 50/20**

(84) Designated Contracting States:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Designated Extension States:
   **BA**
   Designated Validation States:
   **KH MA MD TN**

(71) Applicant: **Koninklijke Philips N.V.**
   **5656 AG Eindhoven (NL)**

(72) Inventors:
   • **MYSORE SIDDU, Dinesh**
     **Eindhoven (NL)**
   • **PAWAR, Anil**
     **Eindhoven (NL)**

   • **ADAK, Saptakatha**
     **5656AG Eindhoven (NL)**
   • **FONOLLA NAVARRO, Roger**
     **Eindhoven (NL)**
   • **GAYET, Maudy**
     **Eindhoven (NL)**
   • **DE BECKER, Jan**
     **Eindhoven (NL)**
   • **Ralf Dieter, HOFFMANN**
     **Eindhoven (NL)**

(74) Representative: **Philips Intellectual Property &
   Standards
   High Tech Campus 52
   5656 AG Eindhoven (NL)**

(54)    **DETECTING AND GRADING REGION OF INTEREST IN 3D IMAGE DATA OF PROSTATE**

(57)    A method for grading a lesion in three-dimensional image data of a prostate of a patient is provided. The method includes registering 3D image data that includes a plurality of different types of MRI images of the patient. A prostate region in the 3D image data is segmented into a region of interest comprising a lesion from which multiple radiomics features are extracted. A lesion is graded by applying a machine learning model to the multiple radiomics features.

EP 4 524 881 A1

FIG. 5B

**Description**

FIELD OF THE INVENTION

The invention relates to a system and method for grading a lesion in three-dimensional image data of a prostate of a patient, a system and method for training a plurality of machine learning models to grade a lesion in three-dimensional image data of a prostate of a patient, and a computer-readable medium.

BACKGROUND OF THE INVENTION

The prostate gland is a critical component of the male reproductive system, and its health is essential for the overall well-being of an individual. Various ailments can affect the prostate, with prostate cancer being one of the most severe and common.

One way to diagnose prostate cancer is ultrasound-guided prostate biopsy, in which multiple samples are taken from different parts of the prostate. Unfortunately, the biopsy procedure on prostates is affected by high false negative and high false positive rates since early-stage prostate cancer (PCa) is generally not visible on ultrasound during the biopsy procedure. As a result, a negative biopsy does not rule out a diagnosis of prostate cancer, might miss out many tumours or might lead to unnecessary biopsies on the patient.

There is therefore a desire to better predict the presence of a tumour in the prostate, and if so, to grade it, e.g., on a scale which indicates the severity of the tumour.

Accurate and early detection of regions of interest (ROI) within the prostate that are suspicious for cancerous lesions is imperative for timely intervention and treatment. In this context, medical imaging is an invaluable tool for non-invasive examination of the prostate.

Conventionally, radiologists have relied on magnetic resonance imaging (MRI) for visualizing the internal structure of the prostate. Among various MRI techniques, T2-weighted (T2W) images have been particularly favoured for detecting regions suspicious for prostate cancer. The T2W images offer enhanced contrast, which makes it easier to discern between normal and abnormal tissues.

There are several challenges when using T2-weighted images for grading prostate cancer. First segmenting the prostate and identifying regions of interest therein is not easy. While automated segmentation systems have been developed, see for example [1], segmenting the prostate gland presents unique challenges. The prostate is a complex organ, with substantial variations in size, shape, and appearance among individuals. Additionally, the prostate's morphology can change due to various factors, including age, disease conditions, and other physiological variables. These factors contribute to a high degree of variability in the size and shape of the prostate gland, which in turn leads to variability in the segmentation of regions of interest in the prostate gland.

Radiologists usually perform the delineation of the region of interest by contouring the prostate in a slice-by-slice manner using different views - axial, sagittal, or coronal, or a combination of these views. This meticulous manual detection process, although effective, is labour-intensive and time-consuming.

In recent times, healthcare systems across the globe have been facing acute shortages in medical staff, which exacerbates the burden on radiologists. Moreover, the manual detection process is subject to human error and variations in interpretation among radiologists. As such, it is highly desirable to develop automated systems that can accurately segment and grade regions of interest within the prostate. Automated detection not only has the potential to relieve the workload on medical staff but also serves as an invaluable second opinion, which can enhance the reliability of the diagnostic process.

Lesion grading by ISUP plays a key role in risk stratification and management of patients with prostate cancer (PCa). Determining a grading such as a Gleason score/ISUP by direct pathologic determination from a tissue specimen is a reliable method, but also invasive and labour intensive.

It is therefore desirable to be able to obtain a better grading for a prostate from medical imaging while handling the varied nature of the prostate gland and produce results with a low number of false positives and negatives, to ensure that the patients receive the most accurate diagnosis and, consequently, the most effective treatment plan.

References

[1] Rouvière O, et al. Combined model-based and deep learning-based automated 3D zonal segmentation of the prostate on T2-weighted MR images: clinical evaluation. Eur Radiol. 2022 May.

**EP 4 524 881 A1**

SUMMARY OF THE INVENTION

In a first aspect of the invention, a computer-implemented method for grading a lesion in three-dimensional [3D] image data of a prostate of a patient is provided, comprising:

- accessing 3D image data, the 3D image data comprising a plurality of different types of MRI images of the patient;
- registering the 3D image data, comprising registering the plurality of different types of MRI images;
- segmenting the registered 3D image data into at least a prostate region by applying a machine learning model to the registered plurality of different types of MRI images;
- further segmenting the prostate region into a region of interest comprising a lesion;
- extracting multiple radiomics features from the region of interest in the plurality of different types of MRI images;
- grading the lesion by applying a machine learning model to the multiple radiomics features.

In an embodiment, the method further comprises normalizing at least part of the plurality of different types of MRI images of the patient by scaling intensity values of anatomical features detected in the plurality of different types of MRI images, e.g., fat and muscle regions. In an embodiment, the segmenting of the registered 3D image data comprises segmenting into at least two glandular regions of the prostate. The glandular regions may be used to extract different radiomics features, e.g., a different set of radiomics features, for different glandular regions and/or for different combinations of glandular region and MRI type.

In a further aspect of the invention, a computer-implemented method is provided for training a plurality of machine learning models to grade a lesion in three-dimensional [3D] image data of a prostate of a patient, comprising:

- accessing training data comprising a plurality of training data instances, wherein a training data instance comprises 3D image data of a prostate of a patient and annotation data which provides an annotation of one or more regions of interest in the 3D image data and a grading, and wherein the 3D image data comprises a plurality of different types of MRI images of the patient;
- providing a plurality of machine learning models, wherein a separate machine learning model is provided for segmenting the registered 3D image data into at least a prostate region, and for further segmenting the prostate region into a region of interest comprising a lesion, and for grading the lesion;
- for each or at least a subset of the training data instances:
- registering the 3D image data, comprising registering the plurality of different types of MRI images;
- segmenting the registered 3D image data into at least a prostate region
- further segmenting the prostate region into a region of interest comprising a lesion;
- extracting multiple radiomics features from the region of interest in the plurality of different types of MRI images;
- grading the lesion by applying a machine learning model to the multiple radiomics features.
- based on said determined prostate region, region of interest, and grading, selecting and training a corresponding machine learning model on the 3D image data using the annotation of the region of interest as prediction target, thereby obtaining a plurality of trained machine learning models;
- outputting model data defining the plurality of trained machine learning models.

In a further aspect of the invention, a grading system is provided. In a further aspect of the invention, a training system is provided. In a further aspect of the invention, a transitory or non-transitory computer-readable medium is provided, the computer-readable medium comprising data representing a computer program comprising instructions for causing a processor system to perform a computer-implemented method as described in this specification.

The above measures involve training a plurality of machine learning models to be able to detect a region of interest in 3D image data of a prostate of a patient and to grade it, and the subsequent use (also referred to as 'inference' in the field of machine learning) of the plurality of machine learning model for the aforementioned detection and grading. This use may for example take place during screening or treatment of a patient.

The training of the machine learning models may involve providing training data which comprises a number of training data instances. Each or at least a subset of these training data instances may comprise 3D image data of a prostate of a patient, for example 3D image data acquired by an MRI system. Interestingly, the 3D image data may provide MRI image data that is obtained from different MRI imaging parameters. In particular, the different types of MRI images may comprise a T2 weighted image and an ADC weighted image.

In addition, annotation data may be provided, which annotation data may provide an annotation of a region of interest in the 3D image data, and if there are multiple regions of interest in the 3D image data, multiple of such annotations. The region of interest may for example be a region which is suspicious for a cancerous lesion or a region which is deemed atypical for other reasons. An annotation may take various forms, such as a segmentation of a region of interest through a delineated outline, or a mask that highlights a specific region or masks out its background. The annotation data may provide any number of such annotations, for example zero (e.g., in case there are no regions of interest in the 3D image data), one, or a plurality of annotations. Such annotations may typically be obtained by manual annotation, for example by a radiologist or other clinical staff. The training data instances may typically relate to different patients. In some examples, multiple training data instances may relate to the same patient, but may differ in terms of 3D image data, for example by having been acquired at different time instances, and/or in annotation data, e.g., by comprising annotations provided by different radiologists.

The training may further involve segmenting the 3D image data of a particular training instance into at least two glandular regions. Such glandular regions may for example include the peripheral region (also called 'peripheral zone') of the prostate and a central gland region of the prostate, with the latter comprising the anterior fibromuscular stroma, the transition zone, and the central zone of the prostate. However, this is not a limitation, in that any other glandular regions may be segmented. Each glandular region may be a 3D region and its segmentation may result in for example a 3D contour or 3D mask. The glandular regions are typically non-overlapping and may together cover at least a part, or in some cases even all of the prostate gland.

The training may further involve providing a separate machine learning model for each of the aforementioned glandular regions. Here, the term 'separate' may refer to the respective models being different instances of a same type of model, e.g., a deep neural network, but with each instance having different trainable model parameters, or the models being of the same type but having a different configuration, e.g., a different number of layers, a different size of the layers, etc., or the models being of a different type, e.g., a deep neural network model and a random forest model.

Having segmented the 3D image data of a particular training data instance, it may be determined which of the at least two glandular regions comprise a region of interest for which an annotation is provided in the annotation data. This may for example involve comparing coordinates of an annotation to coordinates of the segmentation of a glandular region. If one of the glandular regions is determined to comprise a region of interest, the corresponding machine learning model may be trained, e.g., in a training iteration, on the 3D image data of the particular glandular region and using the annotation of the region of interest as a prediction target. This way, the machine learning model may be trained to detect regions of interest in the image data of this particular glandular region. In some examples, if different glandular regions each comprise a region of interest, the corresponding machine learning models may be trained on the respective 3D image data using the respective annotations as prediction targets. In some examples, if there is no region of interest specified in a particular glandular region, for example by the region of interest lying in another glandular region or there not being any region of interest defined by the annotation data, the corresponding machine learning model may still be trained on the 3D image data of the particular glandular region but using a prediction target which represents an absence of a region of interest. In other words, the machine learning model may be trained to refrain from detecting a region of interest in the 3D image data of a particular glandular region. Having segmented regions of interest, radiomics may be derived from them. A machine learning model may be trained as a grader or classifier to map the derived radiomics to a grading result. For example, a grading scale may run from fully indolent, e.g., passive, to fully clinically relevant, e.g., aggressive.

Having trained the plurality of machine learning models on the training data, the trained machine learning models may be deployed in clinical practice. During use ('inference'), 3D image data of a new patient may be segmented into the at least two glandular regions and each machine learning model may be applied to the image data of the corresponding glandular region to obtain a detection result. In other words, if there are two glandular regions for which a trained machine learning model is available, both machine learning models may be applied to the 3D image data of the respective glandular region to produce a detection result. The detection result may take the same or similar form as the annotation data used during training, being for example a contour or a mask. The detection results may then be combined to provide an identification of possible regions of interest for all of the aforementioned glandular regions. It will be appreciated that such a detection result may identify one or more regions of interest but may also indicate that the 3D image data does not contain a region of interest. A grading may be determined for a region of interest that is found.

The above measures have the effect of improving the detection and/or grading of regions of interest in 3D image data of a prostate. This may be explained as follows. The glandular regions have different characteristics. For example, different glandular regions may comprise different types of tissues, for example tissue which comprises secretory acini, muscle tissue, fibrous connective tissue, etc. In addition, the inter-patient variability in the shape and size of each glandular region may be different between glandular regions. As such, regions of interest may have different characteristics in each of the glandular regions. By using separate models for each of the glandular regions, these models may be specialized for the specific characteristics of a region of interest in each glandular region. This specialization results in higher accuracy and performance for each specific detection task, as also demonstrated elsewhere in this specification. Namely, a single model trying to learn the features of multiple glandular regions may become overly complex, as the model has to understand a wide range of features and patterns, and may not perform optimally for all glandular regions as the model's capacity may get diluted in trying to generalize across varied data. By only having to learn the features of regions of interest in a specific glandular region, individual models may be less complex compared to a single, all-encompassing model and provide better performance as the individual models may only need to generalize across one glandular region. It may also be easier to fine-tune or modify a model dedicated to a single glandular region as one does not have to worry about the impact of such fine-tuning on other glandular regions. This allows for optimization focused on the specific challenges of each glandular region. Furthermore, with separate models for each glandular region, performance trade-offs, for example between sensitivity and specificity, may be optimized for each specific glandular region based on its importance and requirements. The use of separate models may also ensure that data imbalances across different glandular regions do not affect each other. Interestingly, while one may expect that when trying to segment different organs, the use of different segmentation techniques may be beneficial, it was found that also the detection of lesions in such regions may benefit from a differentiation between the regions. Most surprisingly, the prostate was found as an extraordinary case where such differentiation within a single organ is very beneficial.

The following embodiments may, unless otherwise noted, relate to each of the training system and corresponding computer-implemented method and the detection and/or grading system and corresponding computer-implemented method, mutatis mutandis.

A method for grading a lesion in three-dimensional image data of a prostate of a patient is provided. The method includes registering 3D image data that includes a plurality of different types of MRI images of the patient. The method may also include normalizing. A prostate region in the 3D image data is segmented into a region of interest comprising a lesion from which multiple radiomics features are extracted. A lesion is graded by applying a machine learning model to the multiple radiomics features.

It will be appreciated by those skilled in the art that two or more of the above-mentioned embodiments, implementations, and/or optional aspects of the invention may be combined in any way deemed useful.

Modifications and variations of any system, any computer-implemented method and/or the computer program product, which correspond to the described modifications and variations of another one of said entities, can be carried out by a person skilled in the art on the basis of the present description.

BRIEF DESCRIPTION OF THE DRAWINGS

These and other aspects of the invention will be apparent from and elucidated further with reference to the embodiments described by way of example in the following description and with reference to the accompanying drawings, in which:

Fig. 1A shows a detection system configured to detect a region of interest in 3D image data of a prostate of a patient using a plurality of machine learning models;
Fig. 1B shows a grading system configured to detect and grade a region of interest in 3D image data of a prostate of a patient using a plurality of machine learning models;
Fig. 1C shows a grading system configured to detect and grade a region of interest in 3D image data of a prostate of a patient using a plurality of machine learning models;
Fig. 2 shows a training system configured to train a plurality of machine learning models to detect a region of interest in 3D image data of a prostate of a patient;
Fig. 3A shows a cross-sectional view of a prostate gland;
Fig. 3B shows a sagittal cross-section of the prostate gland;
Fig. 4 shows an architecture of a nnU-Net machine learning model for segmenting glandular regions in 3D image data of a prostate;
Fig. 5A shows a segmentation of a prostate in an axial image slice;
Fig. 5B shows a segmentation of two glandular regions in the prostate, being a central gland region and a peripheral region;
Fig. 6A shows another example of the segmentation of glandular regions;
Fig. 6B illustrate a segmentation of a glandular region into subregions;
Figs. 7A and 7B each show a comparison between the detection of regions of interest by a machine learning model which is trained to detect regions of interest in the entire prostate region and by a plurality of machine learning models which are specifically trained to each detect regions of interest in a specific glandular region;
Fig. 8 show a T2 image and a corresponding registered ADC image.
Fig. 9 shows a method for grading a lesion in three-dimensional [3D] image data of a prostate of a patient;
Fig. 10 shows a method for training the plurality of machine learning models;
Fig. 11 shows a non-transitory computer-readable medium comprising data.

It should be noted that the figures are purely diagrammatic and not drawn to scale. In the figures, elements which correspond to elements already described may have the same reference numerals.

DETAILED DESCRIPTION OF EMBODIMENTS

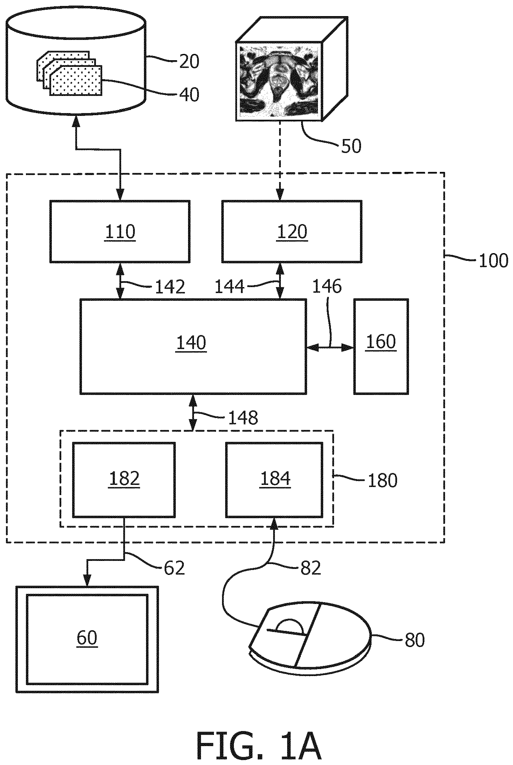

Fig. 1A shows a detection system 100 which is configured for detecting a region of interest in three-dimensional [3D] image data of a prostate of a patient. The detection system 100 is shown to comprise an input interface 120 to access the 3D image data 50. For example, the 3D image data 50 may be accessed via the input interface 120 from a Picture Archiving and Communication System (PACS) or an Electronic Medical Record (EMR) database of a Hospital Information System (HIS) to which the system 100 may be connected or comprised in. The detection system 100 may further comprise a data storage interface 110 to a data storage 20. The data storage 20 may serve as short term and/or long-term data storage. For example, the 3D image data 50 obtained via the input interface 120 may be at least temporarily stored on the data storage 20. In some embodiments, the 3D image data 50 may also be accessed from the data storage 20 instead of using another input interface. In the example of Fig. 1A, the data storage interface 110 is shown to be connected to an external data storage 20. Alternatively, the data storage 20 may be an internal data storage of the detection system 100 (not shown in Fig. 1A). In general, the data storage interface 110 as well as the input interface 120 may take various forms, such as a hard disk or solid-state disk interface to one or more hard disks and/or solid state disks, or a network interface to a Local Area Network (LAN). In some embodiments, the input interface 120 and the data storage interface 110 may be a same interface.

3D image data 50 comprises plurality of different types of MRI images of the patient. Detection system 100 is configured to register the MRI images of different types so that anatomical features align. Having access to registered MRI images of different types improves the accuracy of neural networks, as they have additional information to base segmentation decisions on.

The detection system 100 may further comprise a processor subsystem 140 configured to internally communicate with the input interface 120 via data communication 144, with the data storage interface 110 via data communication 142, with a memory 160 via data communication 146 and with a user interface subsystem 180 via data communication 148. The memory 160 may for example be a volatile memory in which a computer program may be loaded which may cause the processor subsystem 140 to carry out functions which are described in this specification as being performed by the detection system 100.

The user interface subsystem 180 may be an optional component of the detection system 100, and if present, may be configured to, during operation of the detection system 100, enable a user to interact with the detection system 100, for example using a graphical user interface. The user interface subsystem 180 is shown to comprise a user input interface 184 configured to receive user input data 82 from a user input device 80 operable by the user. The user input device 80 may take various forms, including but not limited to a computer mouse, touch screen, keyboard, microphone, etc. Fig. 1A shows the user input device to be a computer mouse 80. In general, the user input interface 184 may be of a type which corresponds to the type of user input device 80, i.e., it may be a thereto corresponding type of user device interface. The user interface subsystem 180 is further shown to comprise a display output interface 182 configured to provide display data 62 to a display 60 to visualize output of the detection system 100. In the example of Fig. 1A, the display is an external display 60. Alternatively, the display may be an internal display.

In some examples, the detection system 100 may comprise the aforementioned display output interface but not comprise a user input interface. In some examples, the detection system 100 may comprise another type of output interface, such as an audio interface, e.g., to a loudspeaker, or a network interface. Any of such output interfaces may be used to render or transmit output generated by the detection system 100.

The processor subsystem 140 may be configured to, during operation of the detection system 100, segment the 3D image data 50 into at least two glandular regions, and for each of the glandular regions, provide a separate machine learning model which is trained to detect a region of interest in the 3D image data of a respective glandular region. For example, the at least two separate machine learning models may be accessed as model data 40 from the data storage 20. The processor subsystem 140 may be further configured to, during operation of the detection system 100, apply the separate machine learning model to the 3D image data of the respective glandular region to obtain a detection result, thereby obtaining a plurality of detection results for the glandular regions, and via an output interface, such as the display output interface 182, output detection data indicative of the plurality of detection results. These and other operations of the detection system 100, and various optional aspects thereof, will be explained in more detail elsewhere in this specification.

In general, the detection system 100 may be embodied as, or in, a single device or apparatus. The device or apparatus may be a general-purpose device or apparatus, such as a workstation or a computer, but may also be an application-specific device or apparatus, such as a radiology display workstation. The device or apparatus may comprise one or more microprocessors which may represent the processor subsystem, and which may execute appropriate software. The software may have been downloaded and/or stored in a corresponding memory, e.g., a volatile memory such as RAM or a nonvolatile memory such as Flash. Alternatively, the functional units of the system, e.g., the input interface, the output interface, and the processor subsystem, may be implemented in the device or apparatus in the form of programmable logic, e.g., as a Field-Programmable Gate Array (FPGA). In general, each functional unit of the detection system 100 may be implemented in the form of a circuit. It is noted that the detection system 100 may also be implemented in a distributed manner, e.g., involving different devices or apparatuses. For example, the distribution may be in accordance with a client-server model, e.g., using a server and workstation. For example, the user input interface and the display output interface may be part of the workstation, while the processor subsystem may be a subsystem of the server. It is noted that various other distributions are equally conceivable.

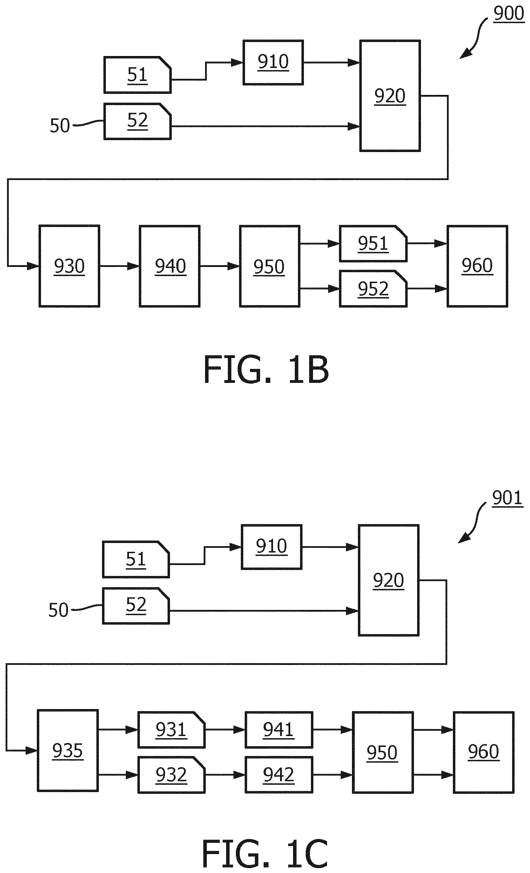

Fig. 1B shows a grading system 900 configured to detect and grade a region of interest in 3D image data of a prostate of a patient using a plurality of machine learning models. Segmenting the 3D image data into a region of interest may use a system such as system 100 for segmenting the image data. System 900 may be integrated into system 100. Other platforms may be used, e.g., as described herein.

System 900 is configured for grading a lesion in three-dimensional [3D] image data of a prostate of a patient. A console or the workstation may be configured as system 900. Grading of a region of interest, especially in the prostate has various applications. For example, an increase in the accuracy with respect to lesion grading may avoid unnecessary prostate biopsies, as better stratification is possible with better gradings. Furthermore, system 900 may provide increased accuracy of grading and thus improved treatments decisions based thereupon.

A particularly interesting application is active surveillance. Active surveillance is a strategy for managing prostate cancers that are considered to be low risk. It involves closely monitoring the patient's condition without giving any immediate treatment. By regularly monitoring the condition, in particular by taking an mpMRI and grading regions of interest in the prostate, a decision can be made to continue active surveillance or to progress to a more invasive treatment. For example, increasing Gleason grades as determined from mpMRI may be an indication to discontinue active surveillance, or to take a biopsy.

The improved accuracy of embodiments may lead to the use of MRI, e.g., mpMRI, as a non-invasive screening tool, meaning that with negative mpMRI biopsy can be omitted, and only patients with a positive mpMRI should receive targeted biopsy to confirm grading.

With the availability of lesion grading, interobserver variability of radiologists can be reduced while assigning scores on the PIRADS scale for the prostate lesions.

Conventional active surveillance protocols exist but are based on PSA values and digital rectal examination (DRE). An MRI grading algorithm may be used to determine when to upgrade, and perform a biopsy in selected patients. In this way, many biopsy procedures and related side effects can be avoided.

System 900 is configured to access 3D image data of a patient. The 3D image data comprises a plurality of different types of MRI images of the patient. For example, the different types may be different types of sequences or imaging protocols. Different types of MRI images highlight and distinguish between different types of tissues or to provide different types of information about the tissues. In an embodiment, the plurality of different types of MRI images are obtained from at least an apparent diffusion coefficient (ADC) sequence and from a T2 signal intensity sequence.

In an embodiment, the plurality of different types of MRI images may comprise multiparametric MRI images (mpMRI). Multiparametric MRI (mpMRI) comprise T2-weighted, diffusion weighted, and dynamic contrast enhanced. Optionally, mpMRI may also comprise MR spectroscopy (MRSI) images.

In an embodiment, multi-typed MRI images are processed through different stages including registration, prostate segmentation, prostate lesion segmentation and Grading of segmented lesions. Preferably, a normalization stage is also included.

Shown in fig. 1B, a T2 weighted MRI image 51 of a prostate and an ADC weighted MRI image 52 of a prostate are accessed. For example, multiple slices of T2 weighted and of ADC weighted MRI images may be obtained. Embodiments may be modified to work with different combinations of MRI image types. For example, in an embodiment an mpMRI image is accessed. For example, in an embodiment, the different types may comprise two or more of: T1-weighted, T2-weighted, Diffusion Weighted Imaging (DWI) with Apparent Diffusion Coefficient (ADC), Dynamic Contrast Enhanced (DCE) sequences, T2* weighted or Gradient echo (GRE) sequences, and Fast Spin Echo (FSE).

The combination of a T2 weighted image 51 and a corresponding ADC weighted image 52 was shown to be particularly successful. This combination will be used in the following exemplary embodiments, but these may be adapted to other combinations by retraining the corresponding machine learned models.

System 900 comprises a registration unit 920. Registration unit 920 is configured to register the plurality of different types of MRI images, in this case to register images 51 and 52. The registration aligns the two or more images of the prostate of different MRI type, e.g., by bringing them into a common coordinate system.

Registering the ADC and T2 image provides a correct alignment of anatomical features between both images. A particular advantageous algorithm is the Fast elastic image registration (feir) algorithm described in "Comparing nonrigid registration techniques for motion corrected MR prostate diffusion imaging", by C. Buerger, J. Sénégas, S. Kabus, H. Carolus, and H. Schulz. Feir is an elastic registration algorithm based on modelling the deformation between the images as the one of an elastic material. Since both images belong to the same subject, an intra-subject registration is performed. Elastic registration is a good choice due to anatomical displacement between correspondent ADC and T2 slices. In an embodiment, the ADC image is chosen as the moving image, e.g., the image to be registered, and T2 as the fixed image. Instead of Feir other conventional registration algorithms may be used.

System 900 comprises a normalization unit 910. Normalization is applied to T2 weighted MRI image 51. Normalization unit 910 normalizes the intensity distribution of voxel values in the T2 weighted MRI image. Normalization helps to support multiple MRI machines, e.g., MRI machines from different manufacturers. As the T2 weighted images of different machines, especially of different makes, the segmentation may for some machines may be less optimal. In an embodiment, the normalization uses a machine learning model, in this case a deep learning model, to detect fat and muscle tissue in the T2 weighted image adjacent to the prostate area. The voxel or pixel values of the fat and muscles tissue may be used as two anchor values to stretch or compress the value range towards a standardized range. For example, the peak, x%-percentile, average, maximum, minimum, etc., value may be used as anchor value.

As shown in fig. 1B, the normalization takes place before registering the images. This is not necessary. The normalization could just as well be done after registration 920. Normalization needs solely the T2 image. However, it is possible to use also the ADC image. Normalization of the T2 image after registration has the advantage that the fat/muscle detecting model may be configured to receive the T2 image, but also the ADC image. Although the ADC image does not need to be normalized, having access to the ADC image helps the model identifying muscle and fat tissue. In an embodiment, system 900 is configured to apply a machine learned model to a registered T2 weighted and ADC weighted image to detect in the T2 weighted image pixels belonging to fat and to muscle respectively. A linear transformation may be used to map the pixels in the unnormalized T2 image to a normalized T2 image, wherein the pixel values of fat and muscle are mapped to predetermined values.

In an embodiment, T2 weighted and ADC weighted MRI images are registered and input to a machine learning model, e.g., a neural network, e.g., a convolutional neural network, to detect in the registered images pixels belonging to fat and to muscle respectively. As the input images are registered it is sufficient for the model to provide output for fat and for muscles only once, e.g., as bounding boxes, or masks. A representative pixel value for the fat and muscles tissues is taken, e.g., the 90-percentile value, the resulting range is then mapped to a standardized range.

A linear transformation may be used to map the pixels in the unnormalized T2 image to a normalized T2 image, wherein the pixel values of fat and muscle are mapped to predetermined values.

In an embodiment, normalization uses a deep learning (DL) model based on the YOLOv5 network trained to detect fat and muscle regions, which are used to obtain peak intensity of both anatomical features, e.g., the highest and lowest peak. The output of the trained network is a set of coordinates as predictions for the bounding boxes. Given a T2 image the deep learning model detects a bounding box or region of interest for both fat and muscle regions. A normalized vendor neutral nT2 image can be calculated applying the following formula:

$$nT2 = \frac{I(x,y,z) - I_{muscle}}{I_{fat} - I_{muscle}}\left(T2_{fat} - T2_{muscle}\right) + T2_{muscle}$$

Where $I(x,y,z)$ is the unnormalized image, $I_{muscle}$ is the 90th percentile intensity of the detected muscle, $I_{fat}$ the 10th percentile intensity of the detected fat, $T2_{fat}$ and $T2_{muscle}$ are the relaxation value of 3T MRI for both fat (121 ms) and muscle (40 ms). More information on tissue normalization can be found in the paper 'Automated reference tissue normalization of T2-weighted MR images of the prostate using object recognition' by Mohammed R. S. Sunoqrot, et al.

Prior to performing the normalization, a bias field correction (BFC) may be performed to remove low-frequency intensity data present on the physical MRI machine. Removing this signal helps to avoid that the same anatomical features may have a different intensity signal, which will affect further normalization steps and segmentations steps. For example, N4 BFC may be method utilized to correct for the bias field in each image. Following the BFC the normalization of the T2 image is calculated.

Typically, system 900 comprises bias field correction followed by registration and normalization, or the other way round. However, in an embodiment, system 900 comprises bias field correction and registration, but not normalization. In an embodiment, system 900 comprises registration but neither bias field correction nor normalization.

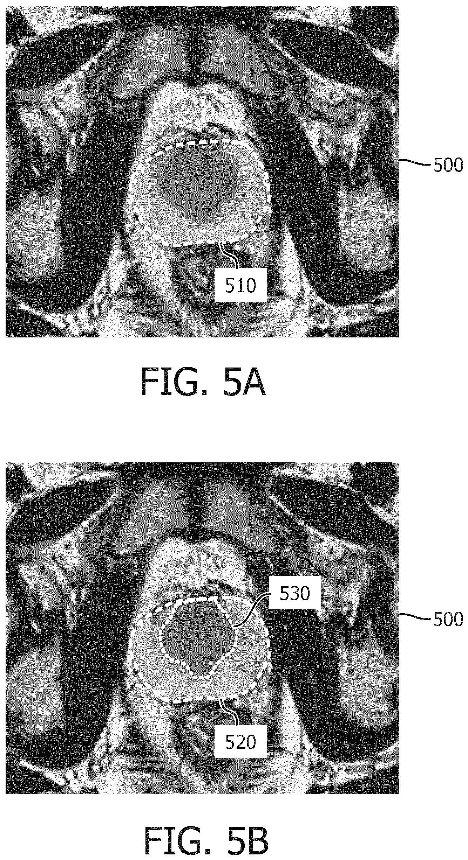

System 900 comprises a first segmentation unit 930. First segmentation unit 930 is configured to segment the registered 3D image data into at least a prostate region, e.g., region 510 in fig. 5A, by applying a machine learning model to the registered MRI images of different types.

First segmentation unit 930 takes as input the 3D image data comprising MRI images of different types, e.g., images 51 and 52, after their preprocessing. The preprocessing includes at least registration, but may also include normalization and/or bias correction. First segmentation unit 930 may comprise one or multiple neural networks comprising one or more convolutional layers. For example, unit 930 may identify the pixels or voxels that belong to the prostate area. Although unit 930 takes two inputs as input, the output needs to be supplied only once, as the inputs are registered.

System 900 comprises a second segmentation unit 940. Second segmentation unit 940 is configured to further segment the prostate region into a region of interest comprising a lesion. There may be more than one region of interest. Second segmentation unit 940 may comprise one or multiple neural networks comprising one or more convolutional layers. Second segmentation unit 940 also takes as input the processed 3D image data comprising MRI images of the different types. Second segmentation unit 940 also receives as input a segmentation of the 3D image data into at least a prostate region. For example, second segmentation unit 940 may receive as input one or more masks that indicate the prostate region in the 3D image data. Second segmentation unit 940 may comprise one or multiple neural networks comprising one or more convolutional layers. For example, unit 940 may identify the pixels or voxels that belong to a lesion area(s).

System 900 comprises a radiomics extraction unit 950 configured to extract multiple radiomics features from the region of interest in MRI images of the different types. For example, multiple radiomic feature routines may be implemented. A radiomic feature routine may receive as input the image data in an identified region of interest and produce a radiomic feature. Interestingly, the radiomic features, and the corresponding routines, may be different for MRI images of different type. In particular, they may be different for T2 weighted and for ADC weighted images.

The radiomics features convert the images into a vector of quantitative values. An algorithm to compute a radiomics feature may analyse the textures, shapes, and intensities within the region of interest. Optionally, one, more or all of the radiomics features may also be computed outside a region of interest, e.g., at the previously identified fat and muscle area, to serve as a comparison value.

Preferably, the radiomics features are Gaussian standardized. For example, a mean value may be subtracted and the result divided by the standard deviation, said mean and standard deviation may be extracted during training from multiple 3D images. Gaussian standardized keeps the features in a particular range to avoid wrong significance of features with higher magnitude of values.

Radiomics features are extracted from lesion regions of both MR T2 weighted and ADC weighted images. Radiomics features may include semantic features like size, shape location, agnostic features like textures based first-order statistics, higher order algorithms like grey level co-occurrence matrices (GLCM), and gray-level run-length matrix (GLRLM). The first-order features are based on statistical information of image which include maximum intensity, minimum intensity, average, standard deviation, variance, energy, entropy, sharpness, skewness, and kurtosis, grayscale, these features can give statistical information based on the frequency distribution of different gray levels in the given image.

Radiomics extraction unit 950 produces two sets of radiomics: T2 -type radiomics 951, and ADC-type radiomics 952, extracted from T2 weighted and ADC weighted image(s) respectively.

The extracted features from both preprocessed T2 and ADC sequences as mentioned above. Extracted feature may be fused together, e.g., in a single vector, before feeding in the next model.

System 900 comprises a grading unit 960. Grading unit 960 is configured to grade region of interest, in particular, the lesion by applying a machine learning model to the multiple radiomics features. Grading unit 960 may grade a region of interest on a scale, e.g., that indicates the severity of the lesion, e.g., how advanced or aggressive a tumour is. The scale may be proprietary scale, e.g., used locally in a hospital. The scale may be a classification, e.g., indolent v/s aggressive. The scale may also be standardized. For example, grading unit 960 may predict a Gleason grade or a Gleason grade group defined by International Society of Urologic Pathologists (ISUP), or a PIRADS score, etc.

In a prototype, machine learning models were trained using extracted radiomics to classify a lesion ROI into clinically significant (Aggressive) vs non clinically significant (Indolent) tumour. A binary classifier for these two classes (clinically significant and non-significant) needs relatively little data. The grading scale can be extended using more training data, e.g., to five classes such as Gleason grade group, GGG: 1-5.

It is expected that the predicted grade contributes to diagnostic accuracy of radiologists. By discriminating benign lesions from cancer and categorizing lesions as indolent v/s aggressive helps in avoiding unnecessary biopsies on the patient.

In an embodiment, the machine learning model for the grading comprises a random forests model. Interestingly, the combination of radiomics and random forests performs well. The inventors realized that this combination needs a relatively small dataset, e.g., compared to training a neural network for the grading, while at the same time being less prone to overfitting.

Indeed, multiple machine learning models were tried to classify indolent and aggressive prostate cancer including Logistic regression, support vector machine (SVM), ensemble methods like

Random Forest, Xgboost and Balanced random forest with 5-fold cross validation. Out of these, random forest had best performance on cross validation set. Moreover, random forest also provided good understandability.

In an embodiment, the following radiomics were used. For the T2 weighted type MRI image: 90Percentile, Difference Entropy, Sum Average, Sum Entropy, Long Run Emphasis, Run Variance, Small Dependence High Gray Level Emphasis, Lesion Volume. And for the ADC weighted image: Mean, Root Mean Squared, Variance, Inverse Variance, Joint Average, Joint Energy, Sum Squares, Gray Level Variance.

In an embodiment, radiomic features were selected from a larger set of potential radiomic features. To reduce overfitting, complexity, training time, and to get a stable and generalized model, feature selection is carried out before model training. For selecting important features, we have used the sequential feature selection (SFS) method. In this algorithm we start with empty set of features and start adding new feature one by one which performs best and improved results along with already selected features. In each step the feature giving maximum improvement is included in the set of selected features. 17 features were selected using the SFS method including 7 from T2 weighted images and 10 from ADC weighted images.

Fig. 1C shows a grading system 901 configured to detect and grade a region of interest in 3D image data of a prostate of a patient using a plurality of machine learning models. System 901 is similar to system 900, except for the first segmentation unit, the second segmentation unit, and optionally the radiomics extraction unit. The choice and/or number of radiomics may be different or the training of radiomics extraction unit 950, but functionally radiomics extraction unit 950 could be the same as in system 900.

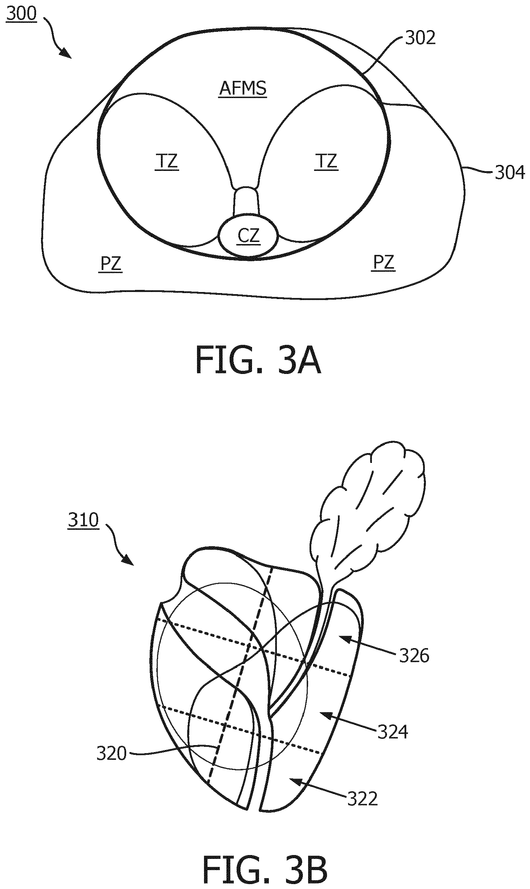

First segmentation unit 935 is similar to unit 930, except that unit 935 is configured to segment the registered 3D image data into at least two glandular regions; e.g., regions 302 and 304 shown in fig. 3A, e.g., a central gland region of the prostate and a peripheral region of the prostate. The at least two glandular regions may comprise a base subregion at a base of the prostate and an apex subregion at an apex of the prostate.

First segmentation unit 935 may receive as input the processed 3D image data, and produce as output multiple regions of the prostate, e.g., as multiple masks. In an embodiment, the prostate segmentation is done in multiple steps. First a whole-prostate machine learning model segments the 3D image data in a prostate region, without further segmenting into glandular regions. A segmented-prostate model may take as input the processed 3D image and the mask produced by the whole-prostate model and produce the masks for the subregions. Segmenting a whole prostate may be easier as more training data is available. Supplying a high quality prostate map to the segmented-prostate model makes the further segmenting task easier. Both the whole-prostate and segmented-prostate model may comprise a neural network, possibly a convolutional network.

System 901 comprises a second segmentation unit for the peripheral region 941 and a second segmentation unit for the central gland region 942. Units 941 and 942 are trained to segment regions of interest, like second segmentation unit 940 but are specialised to do this in the peripheral region and central gland region respectively. For example, units 941 and 942 may receive as input the processed 3D image data, and a mask segmenting the peripheral region and central gland region respectively. The output of each model may be the regions of interest. Regions of interests may be indicated as bounding boxes or as masks.

Radiomics extraction unit 950 may receive the regions of interest from units 941 and 942. Radiomics extraction unit 950 may be the same as in system 900, e.g., extracting radiomics from the T2 and ADC weighted images. In fact in an embodiment, the same radiomics are used as indicated for system 900. In a variant, radiomics extraction unit 950 extracts different radiomics for the T2 and ADC weighted images but also different radiomics for the peripheral region and central gland region. In total four different sets of radiomics may be used for the regions of interest in: T2+ peripheral, ADC+peripheral, T2+central gland, ADC+central gland. All radiomics may be combined, e.g., fused, and passed to grader 960.

In a variant, lesion segmentation may be done on the whole prostate, e.g., using units 930 and 940 but extraction of radiomics may be specific to different zones. A region of interest recognized by unit 940 may be classified into a zone using the segmentation of unit 935. This alternative has the advantage that high-trained third party segmenters may be used for units 930 and 940, while at the same time radiomics may be extracted on a per zone basis, e.g., different radiomics being extracted from different zones and/or different MRI type, e.g., a different set of radiomics for each combination of MRI type and zone. In this case, zone segmentation is only used to utilize the correct radiomic for the zone; here it is acceptable if the segmentation is of slightly lower quality.

Below a list of radiomics for the extended radiomics extraction unit 950 is presented

| Peripheral Zone | | Central Gland | |
|---|---|---|---|
| T2 features | ADC features | T2 features | ADC features |
| 10Percentile | 10Percentile | Entropy | Median |
| Kurtosis | Maj orAxisLength | InterquartileRa nge | Maximum2DDiameterColu mn |
| Flatness | Maximum2DDiameterRo w | Minimum | Sphericity |
| Maximum2DDiameterSlic e | Imc1 | Range | Idmn |
| SurfaceArea | Inverse Variance | RootMeanSqu ared | SmallAreaLowGrayLevelE mphasis |
| SizeZoneNonUniformity | SumEntropy | MajorAxisLen gth | Busyness |

(continued)

| Peripheral Zone | | Central Gland | |
|---|---|---|---|
| T2 features | ADC features | T2 features | ADC features |
| LongRunLowGrayLevelE mphasis | ShortRunLowGrayLevelE mphasis | ClusterPromin ence | Coarseness |
| RunPercentage | Coarseness | ClusterShade | |
| ShortRunEmphasis | DependenceNonUniformit y | GrayLevelVari ance | |
| Contrast | | LongRunEmp hasis | |
| Strength | | | |
| DependenceNonUniformit y | | | |

Below performance on a 5-fold dataset for a Peripheral Zones Lesions Classifier vs a Central Gland Lesions Classifier is shown using an implementation of system 901 and the extended radiomics unit.

| Peripheral Zones Lesions Classifier | | | | | | | |
|---|---|---|---|---|---|---|---|
| Data | No of lesions | Accuracy | F1 Score | Precision | Sensitivity | Specificity | ROC_AUC |
| 5-fold Cross validation | 868 | 69 | 65 | 69 | 62 | 75 | 72 |
| Central Gland Lesions Classifier | | | | | | | |
| Data | No of lesions | Accuracy | F1 Score | Precision | Sensitivity | Specificity | ROC_AUC |
| 5-fold Cross validation | 166 | 79 | 81 | 77 | 87 | 79 | 80 |

For example, in an embodiment, a computer-implemented method (600) for grading a lesion in three-dimensional [3D] image data of a prostate of a patient is provided, comprising:

- accessing (610) 3D image data (50), the 3D image data (50) comprising a plurality of different types of MRI images of the patient, wherein the plurality of different types of MRI images are obtained from at least an apparent diffusion coefficient (ADC) sequence and from a T2 signal intensity sequence;
- normalizing the intensity distribution of values in the MRI image obtained from a T2 signal intensity sequence;
- registering (620) the 3D image data, comprising registering the plurality of different types of MRI images,
- segmenting (630) the registered 3D image data into at least two glandular regions of a prostate region by applying a machine learning model to the registered plurality of different types of MRI images;
- further segmenting (640) the prostate region into a region of interest comprising a lesion;
- extracting (650) multiple radiomics features from the region of interest in at least two MRI images of different type in the plurality of different types of MRI images, wherein the type of radiomics extracted from MRI images depends on the types of MRI image and/or the glandular region in which the region of interest is located,
- grading (660) the lesion by applying a machine learning model to the multiple radiomics features.

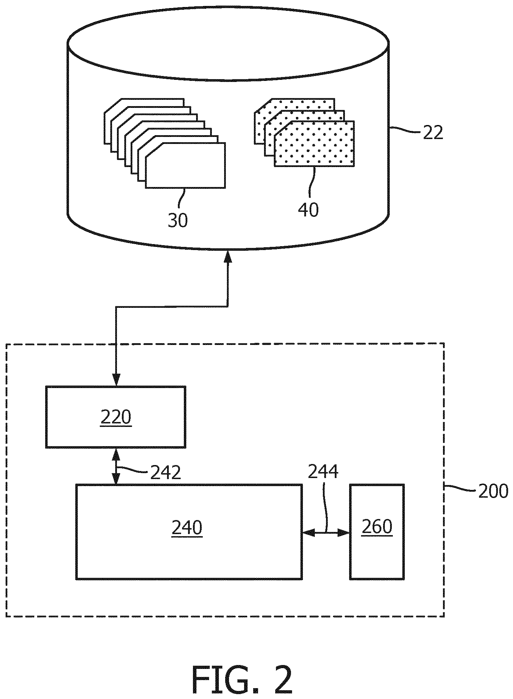

Fig. 2 shows a training system 200 which is configured to train a plurality of machine learning models to detect and grade a region of interest in 3D image data of a prostate of a patient. The plurality of trained machine learning models may then be used by the detection system 100 of Fig. 1A, system 900 of Fig. 1B or system 901 of Fig. 1C to detect and grade regions of interest in new 3D image data, e.g., of a new patient. The training system 200 is shown to comprise an input interface to access training data 30 to train the machine learning model. In the example of Fig. 2, the input interface is shown to be a data storage interface 220 to an external data storage 22 which comprises the training data 30. The data storage 22 and the data storage interface 220 may for example be of a type as described with reference to Fig. 1A and thereby correspond in type to the data storage 20 and the data storage interface 120 of the detection system 100. However, this is not a limitation, in that the input interface 220 of the training system 200 may also be of any other type, such as a network interface to a Local Area Network (LAN) .

The training data 30 may comprise a set of training data instances. Each or at least a subset of the training data instance may comprise 3D image data of a prostate of a patient and annotation data which provides an annotation of one or more regions of interest in the 3D image data and a corresponding grading. In other words, the 3D image data of a prostate and the accompanying annotation(s) may together form a training data instance, and several training data instances, e.g., from different patients, may together form the training data 30.

The training system 200 may further comprise a processor subsystem 240 configured to internally communicate with the input interface 220 via data communication 242 and with a memory 260 via data communication 244. The memory 260 may for example be a volatile memory in which a computer program may be loaded which may cause the processor subsystem 240 to carry out functions which are described in this specification as being performed by the training system. The processor subsystem 240 may be configured to, during operation of the training system 200, provide a plurality of machine learning models, wherein a separate machine learning model is provided for each of at least two glandular regions of the prostate, and for each or at least a subset of the training data instances, segment the 3D image data into the at least two glandular regions, determine which of the at least two glandular regions comprises a region of interest for which an annotation is provided in the annotation data, and based on said determined glandular region, select and train a corresponding machine learning model on the 3D image data using the annotation of the region of interest as prediction target, thereby obtaining a plurality of trained machine learning models. As a result, a plurality of trained machine learning models may be obtained, which may be stored as model data 40, e.g., in the database 22 and which may be transmitted or in any other way distributed, e.g., to the aforementioned detection system 100 of Fig. 1A. These and other operations of the training system 200, and various optional aspects thereof, will be explained in more detail elsewhere in this specification.

In general, the training system 200 may be embodied as, or in, a single device or apparatus, but also in a distributed manner, e.g., involving different devices or apparatuses, and in a same manner as previously described for the detection system 100.

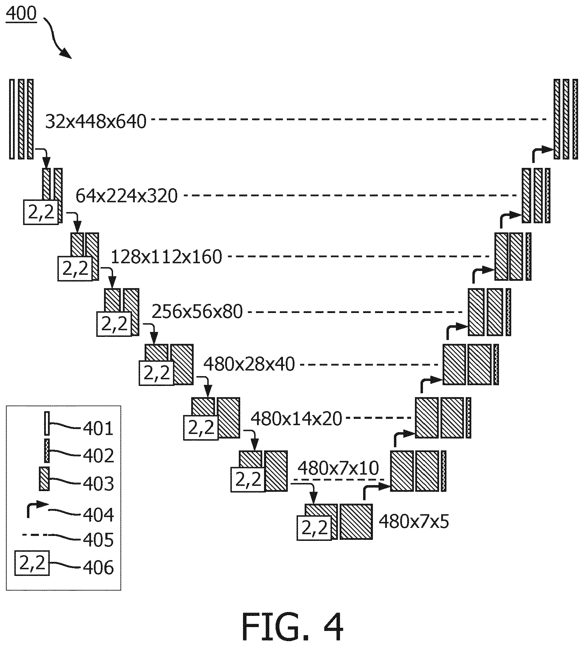

Fig. 3A shows a transverse cross-section 300 of prostate gland, e.g., at 90 degrees to the cranio-caudal axis of the prostate. It can be seen that the prostate gland comprises a plurality of zones, which zones include, but are not limited to, a peripheral zone PZ, a transition zone TZ, a central zone CZ, and an anterior fibromuscular stroma AFMS. The detection system 100 of Fig. 1A and corresponding method may partition the 3D image data of the prostate gland into at least two glandular regions. These glandular regions may later be separately processed by the system and method to detect regions of interests in the respective regions. In the example of Fig. 3A, the segmented regions may comprise a peripheral region 304, which may directly correspond to or comprise the peripheral zone PZ, and a central gland region 302. The central gland region 302 may for example comprise the central zone CZ, the transition zone TZ, and the anterior fibromuscular stroma AFMS. Although not explicitly shown in Fig. 3A, the segmentation may be a 3D segmentation, in that it may yield a 3D delineation of the peripheral region 304 and the central gland region 302, for example in form of respective 3D contours or respective 3D masks. The segmentation of the 3D image data into the peripheral region 304 and the central gland region 302 may be provided by a machine learning model which is trained for this purpose. An example of such a machine learning model is described with reference to Fig. 4.

Fig. 3B shows a sagittal cross-section 310 of the prostate gland, e.g., through the cranio-caudal axis 320 of the prostate, and illustrates that in some embodiments, the glandular regions may be divided along the cranio-caudal axis 320 of the prostate into at least two subregions. In the specific example of Fig. 3B, the subdivision of the glandular regions is shown to be into three subregions, namely an apex region 322 near an apex of the prostate, a base region 326 near a base of the prostate, and a middle region 324 in between the apex region 322 and the base region 326. However, the subdivision along the cranio-caudal axis 320 may also be into two subregions or more than three subregions. In this respect, it is noted that Fig. 3B does not explicitly show the glandular regions as being sub-partitioned but rather illustrates the boundaries of the aforementioned subregions. When partitioning each of the central gland region and the peripheral region into three subregions, six subregions may be obtained, namely a central gland apex region, a central gland middle region, a central gland base region, a peripheral apex region, a peripheral middle region, and a peripheral base region. It will be appreciated, however, that this partitioning is merely exemplary and any other partitioning into glandular regions and sub-partitioning of such glandular regions along the cranio-caudal axis 320 may be used instead.

Fig. 4 shows an architecture 400 of a nnU-Net machine learning model for segmenting glandular regions in 3D image data of a prostate. Here, the term 'nnU-Net' refers to the self-adapting framework for U-Net-based medical image segmentation as described by https://arxiv.org/abs/1809.10486. For example, the architecture may be used for segmenting units 930, and/or 935 and/or further segmenters 940, 941 and/or 942. Also normalizing unit 910 may use the architecture to segment fat and muscle. As is known per se, a nnU-Net machine learning model comprises an encoder, a bottleneck, and a decoder. However, different to conventional applications of nnU-net, the model may receive multiple registered images of different MRI type.

The encoder, which is also referred to as the 'contracting path', is typically composed of blocks, each containing convolutional layers followed by a ReLU activation function and a max-pooling layer for downsampling. This encoder may be used to capture the context of the 3D image data by reducing spatial dimensions while increasing the number of feature channels. The bottleneck, positioned between the encoder and decoder, may comprise convolutional layers to extract dense feature representations. Finally, the decoder may contain blocks with upsampling steps followed by convolutional layers and may restore the spatial dimensions of the feature maps. Skip connections from the encoder may concatenate feature maps to the decoder, helping in reconstruction. Finally, an output layer may provide 1x1 convolution followed by a softmax or sigmoid activation function to, for example, make pixel-wise predictions. Fig. 4 shows a specific example of such an architecture which is optimized for segmenting the glandular regions in a prostate and in which plain-strided convolution layers are used for downsampling and transposed convolution layers are used for upsampling. As can be seen in Fig. 4, the corresponding strided and transposed convolutions are connected through skip connections. With further reference to Fig. 4, it is noted that numeral 401 refers to an input, numeral 402 to a 1x1 convolution with softmax operator, numeral 403 to a 3x3 convolution using an improved ReLu ('IReLu') as activation function, numeral 404 to a transposed convolution, numeral 405 to a skip connection, and numeral 406 to stride (n, n).

In a particular instance, the nnU-Net model underwent training using the Stochastic Gradient Descent (SGD) optimizer. Nesterov momentum, a variant of momentum that adds some future knowledge to the weight updates, was employed with a value of 0.99 to enhance the optimizer's performance. The learning rate, which controls the step size in weight updates, was set at 0.01 and was gradually reduced using a polyLR scheduler - a strategy that adjusts the learning rate according to a polynomial decay. The training was carried out over 600 epochs. The objective of this training was to minimize a hybrid loss function that combines Binary Cross-Entropy Loss and Dice Loss. Binary Cross-Entropy Loss measures the dissimilarity between the true labels and predicted probabilities, making it suitable for binary classification problems. Dice Loss, on the other hand, is specifically tailored for segmentation tasks, as it measures the overlap between the predicted segmentation and the ground truth. By combining these two loss functions, a better performance was obtained for the segmentation of the glandular regions.

In general, the nnU-Net model may be trained on segmentations of glandular regions provided by radiologist or similar types of ground-truth segmentations. In some embodiments, a machine learning model, such as the aforementioned nnU-Net model, may provide a segmentation of glandular regions while the segmentation of a glandular region into subregions may be carried out using heuristics, for example using a simple function which assigns pixels or voxels within a segmented glandular region to a respective subregion based on its coordinate along the cranio-caudal axis. For example, a middle third of the cranio-caudal axis may be assigned to a middle subregion of a respective glandular region, a top-third to an apex subregion of the glandular region, and a bottom-third to a base subregion of the glandular region. In other embodiments, the machine learning model, such as the aforementioned nnU-Net model, may directly provide a segmentation of glandular regions and the corresponding subregions along the cranio-caudal axis.

Fig. 5A shows a segmentation 510 of a prostate in an axial image slice 500, which segmentation may be generated by the aforementioned nnU-Net model. As illustrated in Fig. 5B, the nun-Net model may further segment the prostate into two glandular regions, being in this example the central gland region 530 and the peripheral region 520. Below the dice score performance an nnU-Net model is provided.

| Architecture | Prostate | Central gland zone | Peripheral zone |
|---|---|---|---|
| nnU-Net | 0.922 ± 0.07 | 0.858 ± 0.154 | 0.822 ± 0.79 |

These metrics are Dice scores that evaluate the similarity between a predicted segmentation mask and a ground truth segmentation mask marked by radiologist.

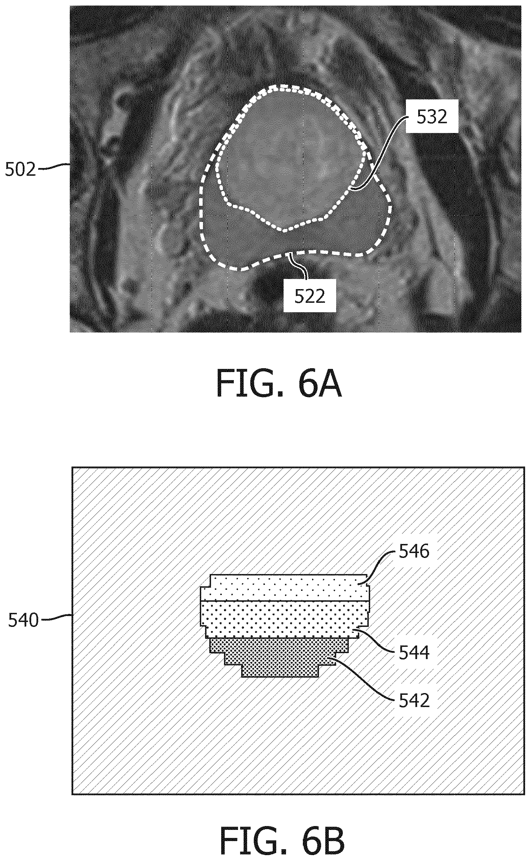

Fig. 6A shows another example of the segmentation of the central gland region 532 and the peripheral region 522 in an axial image slice 502, which segmentation may be generated by the aforementioned nnU-Net model. These glandular regions are then partitioned into subregions. Fig. 6B shows the partitioning into subregions for the whole prostate of Fig. 6A, resulting in the partitioning into a central gland apex region 542, a central gland middle region 544, and a central gland base region 546.

Having segmented the 3D image data of the prostate into two or more glandular regions, and optionally a number of subregions along the cranio-caudal axis, separate machine learning models may be applied to each of these (sub) regions. These machine learning models may for example also be nnU-Net models, e.g., having an architecture as described with reference to Fig. 4 and elsewhere in this specification. Each of the machine learning models may be trained to annotate regions of interest in a respective (sub)region. This annotation may for example involve segmenting or highlighting or in any other way annotating the region of interest. In order to train the respective machine learning models, ground truth data may be used, for example in the form of manual annotations of regions of interest obtained from one or more radiologists. In a specific example, the machine learning models may be trained to detect and segment lesions in the 3D image data of a respective (sub)region. At inference time, each of the machine learning models may be applied to the 3D image data of a respective (sub)region. To ensure that a machine learning model is only or predominately applied to the image data of the respective (sub)region, image data belonging to other (sub)regions may be masked-out or cropped-out or the machine learning model may be otherwise selectively applied to the image data of the respective (sub)region. The detection results from each individual machine learning model may then be combined to obtain a combined detection result for entire prostate.

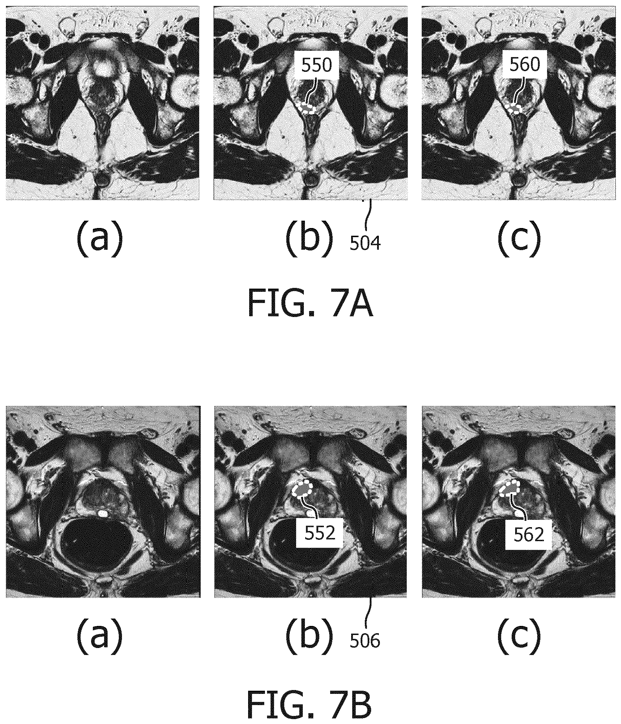

Figs. 7A and 7B each show a comparison between the detection of regions of interest by a machine learning model which is trained to detect regions of interest in the entire prostate region and by a plurality of machine learning models which are specifically trained to each detect regions of interest in different and specific glandular regions. Specifically, in Fig. 7A, (a), (b) and (c) are the qualitative outputs of, respectively, a lesion segmentation model which is trained for the whole prostate, a lesion segmentation model which is trained only for the peripheral region, and the ground-truth annotation. In Fig. 7B, (a), (b) and (c) are the qualitative outputs of, respectively, a lesion segmentation model which is trained for the whole prostate, a lesion segmentation model which is trained only for the central gland region, and the ground-truth annotation. In both figures, it can be seen that a lesion 550, 552 by the respective specialized model while the whole prostate model is unable to detect the lesion. This is confirmed by the recall rate (sensitivity, true positive rate) as indicated below, from which it follows that the use of multiple specialized segmentation models improves upon the use of one segmentation model for the whole prostate.

| Merged recall from individual specialized models | Recall for whole prostate model |
| --- | --- |
| 0.623 | 0.6 |

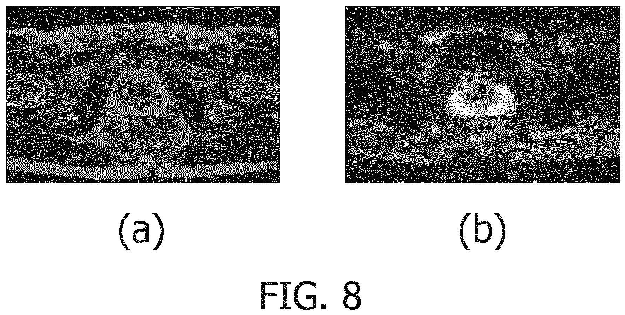

Fig. 8 shows a T2 weighted image and a corresponding registered ADC weighted image. The image at (a) is a T2 weighted image. A corresponding ADC weighted image was obtained from an MRI machine and registered to the T2 image. The resulting registered image is shown at (b). Both images (a) and (b) may be input to a segmenting or further segmenting model. Both images may be used to derive radiomics from any region of interest found.

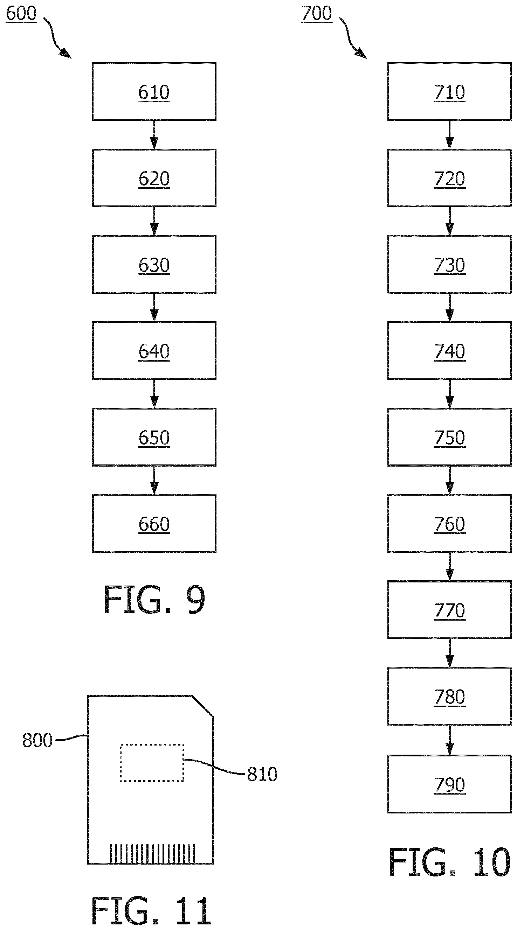

Fig. 9 shows a method 600 for grading a lesion in three-dimensional [3D] image data of a prostate of a patient. The method may be computer implemented. Method 600 comprises:

- accessing (610) 3D image data (50), the 3D image data (50) comprising a plurality of different types of MRI images of the patient;
- registering (620) the 3D image data, comprising registering the plurality of different types of MRI images;
- segmenting (630) the registered 3D image data into at least a prostate region (302, 304) by applying a machine learning model to the registered plurality of different types of MRI images;
- further segmenting (640) the prostate region into a region of interest comprising a lesion;
- extracting (650) multiple radiomics features from the region of interest in the plurality of different types of MRI images;
- grading (660) the lesion by applying a machine learning model to the multiple radiomics features.

Segmenting (630) of the registered 3D image may be into at least two different glandular zones
Fig. 10 shows a method 700 for training a plurality of machine learning models to grade a lesion in three-dimensional [3D] image data of a prostate of a patient. Method 700 may be computer implemented. Method 700 comprises:

- accessing (710) training data (30) comprising a plurality of training data instances,

wherein a training data instance comprises 3D image data of a prostate of a patient and annotation data which provides an annotation of one or more regions of interest in the 3D image data and a grading, and wherein the 3D image data (50) comprises a plurality of different types of MRI images of the patient;

- providing (720) a plurality of machine learning models (40), wherein a separate machine learning model is provided for segmenting the registered 3D image data into at least a prostate region, and for further segmenting the prostate region into a region of interest comprising a lesion, and for grading the lesion;
- for each or at least a subset of the training data instances:
- registering (730) the 3D image data, comprising registering the plurality of different types of MRI images;
- segmenting (740) the registered 3D image data into at least a prostate region (302, 304),
- further segmenting (750) the prostate region into a region of interest comprising a lesion;
- extracting (760) multiple radiomics features from the region of interest in the plurality of different types of MRI images;
- grading (770) the lesion by applying a machine learning model to the multiple radiomics features.
- based on said determined prostate region, region of interest, and grading, selecting and training (780) a corresponding machine learning model on the 3D image data using the annotation of the region of interest as prediction target, thereby obtaining a plurality of trained machine learning models;
- outputting (790) model data defining the plurality of trained machine learning models.

It will be appreciated that steps of the method 600 of Fig. 9 and the method 700 of Fig. 10 may be performed in any suitable order, e.g., consecutively, simultaneously, or a combination thereof, subject to, where applicable, a particular order being necessitated, e.g., by input/output relations. Steps may also be performed as part of other steps.

Any method described in this specification may be implemented on a computer as a computer-implemented method, as hardware, or as a combination of both. As also illustrated in Fig. 11, instructions for the computer, e.g., executable code, may be stored on a computer readable medium 800, e.g., in the form of a series 810 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, etc. Fig. 11 shows a memory device 800.

Examples, embodiments or optional features, whether indicated as non-limiting or not, are not to be understood as limiting the invention as claimed.

The following clauses represent advantageous embodiments.

Clause 1. A computer-implemented method for detecting a region of interest in three-dimensional [3D] image data of a prostate of a patient, comprising:

- accessing the 3D image data;
- segmenting the 3D image data into at least two glandular regions (302, 304);
- for each of the glandular regions:

    - providing a separate machine learning model which is trained to detect a region of interest in the 3D image data of a respective glandular region,
    - applying the separate machine learning model to the 3D image data of the respective glandular region to obtain a detection result (550, 552),

    thereby obtaining a plurality of detection results for the glandular regions; and
- outputting detection data indicative of the plurality of detection results.

Clause 2. The method according to clause 1, wherein the at least two glandular regions include a peripheral region (304) of the prostate and a central gland region (302) of the prostate.

Clause 3. The method according to clause 1 or 2, wherein the central gland region (302) comprises an anterior fibromuscular stroma (AFMS), a transition zone (TZ), and a central zone (CZ) of the prostate.

Clause 4. The method according to any one of clauses 1 to 3, further comprising segmenting each of the at least two glandular regions into at least two subregions (322-326, 542-546) which divide a respective glandular region along a cranio-caudal axis (320) of the prostate, wherein a separate machine learning model is applied to the image data of each of the at least two subregions.

Clause 5. The method according to clause 4, wherein the at least two subregions (322-326, 542-546) comprise a base subregion (326, 546) at a base of the prostate and an apex subregion (322, 542) at an apex of the prostate.

Clause 6. The method according to clause 5, further comprising segmenting each of the at least two glandular regions along the cranio-caudal axis into at least three subregions (322-326, 542-546), wherein the at least three subregions comprise the base subregion, the apex subregion, and a middle subregion (324, 544) positioned between the base subregion and the apex subregion along the cranio-caudal axis.

Clause 7. The method according to any one of clauses 4 to 6, wherein the segmentation into glandular regions (302, 304) is performed using a machine learning model and wherein the segmentation into subregions is performed using heuristics.

Clause 8. The method according to any one of clauses 1 to 7, wherein applying the separate machine learning model to the 3D image data of the respective glandular region (302, 304) comprises masking-out or cropping-away image data outside of the respective glandular region.

Clause 9. The method according to any one of clauses 1 to 8, wherein the 3D image data (50) is obtained by magnetic resonance imaging, for example using a T2 sequence.

Clause 10. The method according to any one of clauses 1 to 9, wherein a respective machine learning model is a neural network comprising one or more convolutional layers.

Clause 11. A computer-implemented method for training a plurality of machine learning models to detect a region of interest in three-dimensional [3D] image data of a prostate of a patient, comprising:

- accessing training data (30) comprising a plurality of training data instances, wherein a training data instance comprises 3D image data of a prostate of a patient and annotation data which provides an annotation of one or more regions of interest in the 3D image data;
- providing a plurality of machine learning models, wherein a separate machine learning model is provided for each of at least two glandular regions of the prostate;

- for each or at least a subset of the training data instances:
- segmenting the 3D image data into the at least two glandular regions (302, 304),
- determining which of the at least two glandular regions comprises a region of interest for which an annotation is provided in the annotation data, and
- based on said determined glandular region, selecting and training a corresponding machine learning model on the 3D image data using the annotation of the region of interest as prediction target, thereby obtaining a plurality of trained machine learning models;
- outputting model data defining the plurality of trained machine learning models.

Clause 12. A transitory or non-transitory computer-readable medium (800) comprising data (810) representing a computer program, the computer program comprising instructions for causing a processor system to perform the method according to any one of clauses 1 to 11.

Clause 13. A detection system (100) for detecting a region of interest in three-dimensional [3D] image data of a prostate of a patient, comprising:

- an input interface (120) for accessing the 3D image data;
- a processor subsystem (140) configured to:
- segment the 3D image data into at least two glandular regions;
- for each of the glandular regions:

    - provide a separate machine learning model (40) which is trained to detect a region of interest in the 3D image data of a respective glandular region,
    - apply the separate machine learning model to the 3D image data of the respective glandular region to obtain a detection result,
    thereby obtaining a plurality of detection results for the glandular regions; and

- an output interface (182) for outputting detection data indicative of the plurality of detection results.

Clause 14. The detection system (100) according to clause 14, wherein the output interface is a display output interface (182) for visualizing the plurality of detection results on a display (60), for example as an overlay over the 3D image data.

Clause 15. A training system (200) for training a plurality of machine learning models to detect a region of interest in three-dimensional [3D] image data of a prostate of a patient, comprising:

- an input interface (220) for accessing training data (30) comprising a plurality of training data instances, wherein a training data instance comprises 3D image data of a prostate of a patient and annotation data which provides an annotation of one or more regions of interest in the 3D image data;
- a processor subsystem (240) configured to:
- provide a plurality of machine learning models (40), wherein a separate machine learning model is provided for each of at least two glandular regions of the prostate
- for each or at least a subset of the training data instances:
- segment the 3D image data into the at least two glandular regions,
- determine which of the at least two glandular regions comprises a region of interest for which an annotation is provided in the annotation data, and
- based on said determined glandular region, select and train a corresponding machine learning model on the 3D image data using the annotation of the region of interest as prediction target, thereby obtaining a plurality of trained machine learning models; and
- an output interface (220) for outputting model data defining the plurality of trained machine learning models.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

List of reference numbers

[0123]    The following list of reference numbers for figures 1a-8, and 11 is provided for facilitating the interpretation of the drawings and shall not be construed as limiting the claims.

AFMS    anterior fibromuscular stroma
CZ       central zone
PZ       peripheral zone
TZ       transition zone

20, 22   data storage
30       training data
40       machine learning models
50       3D image data of prostate
51       a T2 weighted MRI image of a prostate
52       an ADC weighted MRI image of a prostate

60       display
62       display data
80       user input device
82       user input data

100      detection system
110      data storage interface
120      input interface
140      processor subsystem
142-148  data communication
160      memory
180      user interface subsystem
182      display output interface
184      user input interface

200      training system
220      data storage interface
240      processor subsystem
242, 244 data communication
260      memory

300      transverse cross-section of prostate gland
302      central gland region
304      peripheral region
310      sagittal cross-section of prostate gland
320      cranio-caudal axis
322      apex
324      middle
326      base

400      nnU-Net architecture
401      input
402      1x1 convolution - softmax
403      3x3 convolution - IReLu
404      transposed convolution
405      skip connection
406      stride (n, n)

| 500-506 | axial image slice |
| 510 | segmentation of prostate |
| 520, 522 | segmentation of peripheral region |
| 530, 532 | segmentation of central gland region |
| 540 | cranio-caudal axis view |
| 542 | apex region |
| 544 | middle region |
| 546 | base region |
| 550, 552 | detected region of interest |
| 560, 562 | ground truth region of interest |
| | |
| 800 | non-transitory computer-readable medium |
| 810 | data representing computer program |
| | |
| 900 | a grading system |
| | |
| 901 | a grading system |
| | |
| 910 | a normalization unit |
| | |
| 920 | a registration unit |
| | |
| 930, 935 | a first segmentation unit |
| | |
| 931 | a peripheral region segmentation of the prostate |
| | |
| 932 | a central gland region segmentation of the prostate |
| | |
| 940 | a second segmentation unit |
| | |
| 941 | a second segmentation unit for the peripheral region |
| | |
| 942 | a second segmentation unit for the central gland region |
| | |
| 950 | a radiomics extraction unit |
| | |
| 951 | T2 -type radiomics |
| | |
| 952 | ADC-type radiomics |
| | |
| 960 | a grading unit |

**Claims**

1. A computer-implemented method (600) for grading a lesion in three-dimensional [3D] image data of a prostate of a patient, comprising:

   - accessing (610) 3D image data (50), the 3D image data (50) comprising a plurality of different types of MRI images of the patient;
   - registering (620) the 3D image data, comprising registering the plurality of different types of MRI images;
   - segmenting (630) the registered 3D image data into at least a prostate region (302, 304) by applying a machine learning model to the registered plurality of different types of MRI images;
   - further segmenting (640) the prostate region into a region of interest comprising a lesion;
   - extracting (650) multiple radiomics features from the region of interest in the plurality of different types of MRI images;
   - grading (660) the lesion by applying a machine learning model to the multiple radiomics features.

2. The method according to Claim 1, wherein the plurality of different types of MRI images are obtained from at least an apparent diffusion coefficient (ADC) sequence and from a T2 signal intensity sequence.

3. The method according to any of the preceding claims, wherein different types of radiomics features are extracted from the plurality of different types of MRI images, together forming the multiple radiomics features, and wherein the radiomics features are Gaussian standardized.

4. The method according to any of the preceding claims, wherein the machine learning model for grading the lesion comprises a random forests model, and
wherein segmenting (630) the registered 3D image data into at least a prostate region (302, 304) comprises segmenting into at least two glandular regions (302, 304), wherein the further segmenting comprises applying separate machine learning models (40) to each of the segmented glandular regions, the separate machine learning models being trained to detect a lesion in the respective glandular regions.

5. The method according to Claim 4, wherein segmenting into at least two glandular regions comprises segmenting into a prostate region by a whole-prostate machine learning model followed by segmenting the segmented prostate region into at least two glandular regions by a glandular machine learning model.

6. The method according to Claim 5, wherein the glandular machine learning model takes as input at least a prostate region mask produced by the whole-prostate machine learning model, and the registered plurality of different types of MRI images.

7. The method according to any of claims 4-6, wherein

   - the at least two glandular regions include a peripheral region (304) of the prostate and a central gland region (302) of the prostate, and/or
   - the at least two glandular regions (322-326, 542-546) comprise a base subregion (326, 546) at a base of the prostate and an apex subregion (322, 542) at an apex of the prostate.

8. The method (600) according to any of the preceding claims, wherein the extracting of multiple radiomics features from the region of interest in MRI images of the different types comprises

   - extracting different radiomic features for at least two, or for each, of the different types of MRI images in the plurality of different types of MRI images, and/or
   - extracting different radiomic features for at least two, or for each, segmented glandular region in the prostate region, and/or
   - extracting different radiomic features for at least two combinations or for each combination of a segmented glandular region in the prostate region and an MRI type of the plurality of different types of MRI images.

9. The method (600) according to any of claims 1 to 8, wherein the machine learning model for segmenting and/or further segmenting the registered 3D image data comprises one or multiple neural networks comprising one or more convolutional layers.

10. The method (600) according to any of claims 1 to 9, wherein

    - the plurality of different types of MRI images comprises a T2 weighted MRI image, and the multiple radiomics features for the T2 weighted MRI image comprise one or more of: 90Percentile, Difference Entropy, Sum Average, Sum Entropy, Long Run Emphasis, Run Variance, Small Dependence High Gray Level Emphasis, Lesion Volume, and/or
    - the plurality of different types of MRI images comprises an ADC weighted MRI image, and the multiple radiomics features for the ADC weighted MRI image comprise one or more of: Mean, Root Mean Squared, Variance, Inverse Variance, Joint Average, Joint Energy, Sum Squares, Gray Level Variance.

11. The method (600) according to any of claims 1 to 10, wherein the plurality of different types of MRI images comprises a T2 weighted MRI image, the method comprising normalizing the intensity distribution of values in the T2 weighted MRI image before segmenting the registered 3D image data and/or before further segmenting the prostate region into a region of interest.

12. A computer-implemented method (700) for training a plurality of machine learning models to grade a lesion in three-dimensional [3D] image data of a prostate of a patient, comprising:

- accessing (710) training data (30) comprising a plurality of training data instances, wherein a training data instance comprises 3D image data of a prostate of a patient and annotation data which provides an annotation of one or more regions of interest in the 3D image data and a grading, and wherein the 3D image data (50) comprises a plurality of different types of MRI images of the patient;

- providing (720) a plurality of machine learning models (40), wherein a separate machine learning model is provided for segmenting the registered 3D image data into at least a prostate region, and for further segmenting the prostate region into a region of interest comprising a lesion, and for grading the lesion;
- for each or at least a subset of the training data instances:
- registering (730) the 3D image data, comprising registering the plurality of different types of MRI images;
- segmenting (740) the registered 3D image data into at least a prostate region (302, 304),
- further segmenting (750) the prostate region into a region of interest comprising a lesion;
- extracting (760) multiple radiomics features from the region of interest in the plurality of different types of MRI images;
- grading (770) the lesion by applying a machine learning model to the multiple radiomics features.
- based on said determined prostate region, region of interest, and grading, selecting and training (780) a corresponding machine learning model on the 3D image data using the annotation of the region of interest as prediction target, thereby obtaining a plurality of trained machine learning models;
- outputting (790) model data defining the plurality of trained machine learning models.

13. A grading system (900) for grading a lesion in three-dimensional [3D] image data of a prostate of a patient, comprising:

- an input interface (120) for accessing (610) 3D image data (50), the 3D image data (50) comprising a plurality of different types of MRI images of the patient;
- a processor subsystem (140) configured to:
- registering the 3D image data, comprising registering the plurality of different types of MRI images;
- segmenting (630) the registered 3D image data into at least a prostate region (302, 304) by applying a machine learning model to the registered plurality of different types of MRI images;
- further segmenting the prostate region into a region of interest comprising a lesion;
- extracting multiple radiomics features from the region of interest in the plurality of different types of MRI images;
- grading the lesion by applying a machine learning model to the multiple radiomics features, and
- an output interface (182) for outputting grading data indicative of a grading result.

14. The grading system (100) according to claim 13, wherein the output interface is a display output interface (182) for visualizing the region of interest and/or the grading result, for example as an overlay over the 3D image data.

15. A training system (200) for training a plurality of machine learning models to detect a region of interest in three-dimensional [3D] image data of a prostate of a patient, comprising:

- an input interface (220) for accessing (710) training data (30) comprising a plurality of training data instances, wherein a training data instance comprises 3D image data of a prostate of a patient and annotation data which provides an annotation of one or more regions of interest in the 3D image data and a grading, and wherein the 3D image data (50) comprises a plurality of different types of MRI images of the patient;
- a processor subsystem (240) configured to:
- providing (720) a plurality of machine learning models (40), wherein a separate machine learning model is provided for segmenting (630) the registered 3D image data into at least a prostate region, and for further segmenting the prostate region into a region of interest comprising a lesion, and for grading the lesion;
- for each or at least a subset of the training data instances:
- registering the 3D image data, comprising registering the plurality of different types of MRI images;
- segmenting (730) the registered 3D image data into at least a prostate region (302, 304),
- further segmenting the prostate region into a region of interest comprising a lesion;
- extracting multiple radiomics features from the region of interest in the plurality of different types of MRI images;
- grading the lesion by applying a machine learning model to the multiple radiomics features.
- based on said determined prostate region, region of interest, and grading, selecting and training (750) a corresponding machine learning model on the 3D image data using the annotation of the region of interest as prediction target, thereby obtaining a plurality of trained machine learning models;

- an output interface (220) for outputting model data defining the plurality of trained machine learning models.

FIG. 1A

FIG. 1B

FIG. 1C

FIG. 2

300

302

AFMS

TZ          TZ

CZ

PZ          PZ

304

# FIG. 3A

310

326

324

320

322

# FIG. 3B

FIG. 4

500

510

## FIG. 5A

530

500

520

## FIG. 5B

502

532

522

FIG. 6A

540

546

544

542

FIG. 6B

(a)　　　　　(b) 504　　　　　(c)

FIG. 7A

(a)　　　　　(b) 506　　　　　(c)

FIG. 7B

(a)  (b)

FIG. 8

600

610

620

630

640

650

660

FIG. 9

700

710

720

730

740

750

760

770

780

790

FIG. 10

800

810

FIG. 11

| | Europäisches Patentamt European Patent Office Office européen des brevets | **EUROPEAN SEARCH REPORT** | Application Number EP 23 19 6946 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/005931 A1 (MADABHUSHI ANANT [US] ET AL) 2 January 2020 (2020-01-02) | 1-3,8-15 | INV. G06T7/00 |
| Y | * paragraph [0026] - paragraph [0066]; figures 1, 7, 11, 13 * | 4-7 | G06T7/11 G16H30/40 G16H50/20 |
| Y | US 2023/252633 A1 (RIX ANTONY WILLIAM [GB]) 10 August 2023 (2023-08-10) | 4-7 | |
| A | * paragraph [0044] - paragraph [0072]; figures 1-3 * | 1-3,8-15 | |
| A | WO 2022/099303 A1 (UNIV CALIFORNIA [US]) 12 May 2022 (2022-05-12) * paragraph [0055] - paragraph [0070] * | 1-15 | |
| A | KHAN ZIA ET AL: "Recent Automatic Segmentation Algorithms of MRI Prostate Regions: A Review", IEEE ACCESS, IEEE, USA, vol. 9, 21 June 2021 (2021-06-21), pages 97878-97905, XP011866824, DOI: 10.1109/ACCESS.2021.3090825 * abstract * | 1-15 | TECHNICAL FIELDS SEARCHED (IPC) G06T G16H |

The present search report has been drawn up for all claims

| Place of search | Date of completion of the search | Examiner |
|---|---|---|
| The Hague | 21 February 2024 | Lauritzen, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 23 19 6946

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2024

| Patent document cited in search report | | | Publication date | Patent family member(s) | | | Publication date |
|---|---|---|---|---|---|---|---|
| US | 2020005931 | A1 | 02-01-2020 | US | 2020000396 | A1 | 02-01-2020 |
| | | | | US | 2020005931 | A1 | 02-01-2020 |
| US | 2023252633 | A1 | 10-08-2023 | EP | 4211647 | A1 | 19-07-2023 |
| | | | | GB | 2594759 | A | 10-11-2021 |
| | | | | JP | 2023541646 | A | 03-10-2023 |
| | | | | US | 2023252633 | A1 | 10-08-2023 |
| | | | | WO | 2022053817 | A1 | 17-03-2022 |
| WO | 2022099303 | A1 | 12-05-2022 | US | 2023410301 | A1 | 21-12-2023 |
| | | | | WO | 2022099303 | A1 | 12-05-2022 |

For more details about this annex : see Official Journal of the European Patent Office, No. 12/82

**EP 4 524 881 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ROUVIÈRE O et al.** Combined model-based and deep learning-based automated 3D zonal segmentation of the prostate on T2-weighted MR images: clinical evaluation. *Eur Radiol.*, May 2022